# FASCICULE DE BREVET EUROPEEN

(11) **EP 1 817 402 B1**
(45) Date de publication et mention de la délivrance du brevet: **04.06.2008**
(21) Numéro de dépôt: 05823016.0
(22) Date de dépôt: 28.11.2005
(51) Int. Cl.: C12M 1/24

(54) **FLACON DE PREPARATION D'UNE SUSPENSION CYTOLOGIQUE**
KOLBEN ZUR HERSTELLUNG EINER ZYTOLOGISCHEN SUSPENSION
FLASK FOR PREPARING A CYTOLOGICAL SUSPENSION

(30) Priorité: 30.11.2004 FR 0412710
(43) Date de publication de la demande: 15.08.2007
(73) Titulaire: Peltier, Eric, 92140 Clamart (FR)
(72) Inventeur: Peltier, Eric, 92140 Clamart (FR)
(74) Mandataire: Domenego, Bertrand
(86) Numéro de dépôt international: PCT/FR2005/002953
(87) Numéro de publication internationale: WO 2006/058989

(56) Documents cités:
- EP-A- 1 044 652
- US-A- 5 422 273
- US-A- 6 063 038

## Description

La présente invention concerne un flacon de préparation d'une suspension cytologique à base de fixateur.

De tels flacons sont utilisés dans l'état de la technique, pour préparer des suspensions cytologiques notamment cervicales ou vaginales à analyser, etc...

Les prélèvements notamment cervicaux ou vaginaux sont effectués par des praticiens à l'aide de brosses spécifiques fixées par exemple de façon détachable sur des manches de manipulation.

Une fois le prélèvement effectué, le praticien plonge la brosse dans le flacon et détache celle-ci du manche, de manière à permettre aux cellules prélevées de se déposer dans le fixateur.

Cependant, des composants indésirables peuvent également se déposer dans le fixateur, comme par exemple, des débris récupérés par la brosse lors du prélèvement (mucus, agrégations, etc...), des squames issus du praticien et qui se déposent dans le flacon, notamment lors de la manipulation de la brosse pour la détacher du manche, etc..

Or, ces composants peuvent être très gênants lors de l'analyse ultérieure de la suspension.

Le demandeur a déjà proposé une structure particulière de flacon pour tenter de résoudre ces problèmes.

Ce flacon est décrit dans le document FR-A-2 792 331.

Selon ce document, ce flacon est muni d'une ouverture destinée à recevoir une brosse de prélèvement cytologique, fixée de façon détachable sur un manche de manipulation, et est caractérisé en ce que l'ouverture du flacon comporte des moyens de butée pour la brosse, permettant de bloquer celle-ci dans le flacon et de la détacher du manche et au moins une portion de voile ajourée de filtrage de la suspension lors d'un versement.

Cependant, à l'usage, un tel flacon a également présenté un certain nombre d'inconvénients notamment au niveau du colmatage de la portion de voile ajourée de filtrage de la suspension par les composants mentionnés précédemment.

Le but de l'invention est donc de perfectionner encore ces flacons de préparation.

A cet effet, l'invention a pour objet un flacon de préparation d'une suspension cytologique à base de fixateur, équipé de moyens de filtrage au moins en partie immergés dans la suspension, caractérisé en ce que les moyens de filtrage se présentent sous la forme d'un voile de matériau filtrant, formant panier, dont la périphérie est fixée sur le flacon et dont le centre est raccordé à un tube, s'étendant en direction de l'ouverture du flacon, associé à des moyens de maintien en position dans le flacon et adapté pour permettre le passage d'une pipette d'aspiration de la suspension.

Suivant d'autres caractéristiques de l'invention :
- la partie supérieure du flacon est adaptée pour recevoir un couvercle ;
- le couvercle et la partie correspondante du flacon comprennent des moyens de vissage complémentaires ;
- le couvercle comporte en regard de l'extrémité correspondante du tube, au moins une portion perçable et auto-cicatrisante, de passage de la pipette d'aspiration, et adaptée pour venir en appui contre cette extrémité du tube afin d'assurer l'étanchéité entre ce tube et le reste du flacon ;
- la partie inférieure du flacon comporte des moyens formant cône de décantation ;
- les moyens de maintien du tube dans le flacon comportent des bras de maintien en position, s'étendant entre le flacon et le tube ;
- l'un des bras comporte des moyens de butée pour permettre de détacher une brosse de prélèvement d'un manche de manutention afin que celle-ci tombe dans le panier ; et
- l'un des bras comporte des picots de peignage d'une brosse de prélèvement pour faire tomber les éléments prélevés par celle-ci dans le panier.

L'invention sera mieux comprise à la lecture de la description qui va suivre, donnée uniquement à titre d'exemple et faite en se référant aux dessins annexés, sur lesquels :
- la Fig.1 représente une vue schématique en coupe de côté d'un flacon de préparation selon l'invention ;
- la Fig.2 représente une vue schématique partielle de dessus d'un tel flacon ; et
- la Fig.3 représente une vue schématique partielle en coupe de côté d'une portion d'un flacon de préparation selon l'invention.

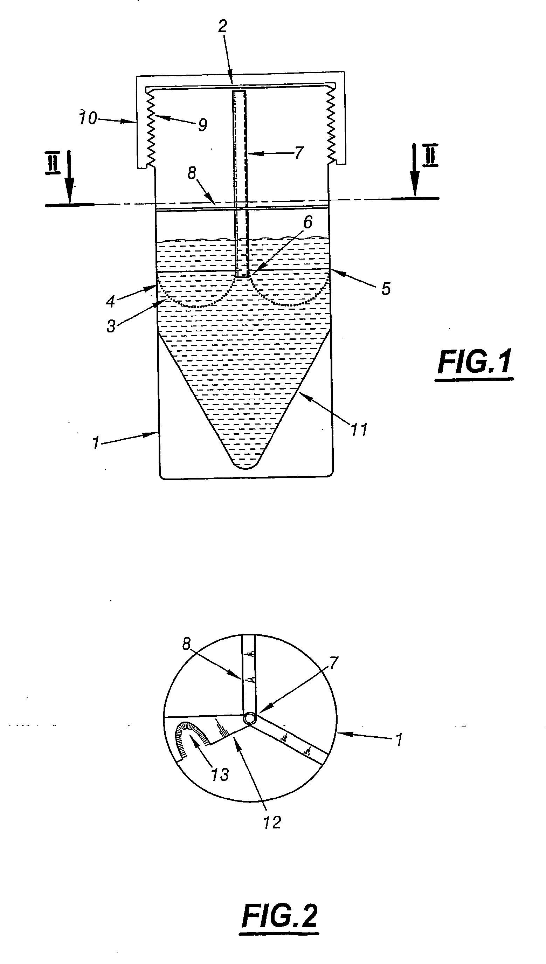

On a en effet illustré sur les figures 1 et 2, un exemple de réalisation d'un flacon selon l'invention.

Ce flacon est un flacon de préparation d'une suspension cytologique à base de fixateur.

Ce flacon est désigné par la référence générale 1 et comporte donc une ouverture désignée par la référence générale 2, cette ouverture permettant le passage d'une brosse de prélèvement cytologique.

Ce flacon est équipé de moyens de filtrage au moins en partie immergés dans la suspension.

Ces moyens de filtrage sont désignés par la référence générale 3 sur cette figure et se présentent, selon l'invention, sous la forme d'un voile de matériau filtrant formant panier désigné par la référence générale 4, dont la périphérie 5 est fixée sur le flacon et dont le centre 6, est raccordé à un tube 7, s'étendant en direction de l'ouverture 2 du flacon, associé à des moyens de maintien en position par exemple centrale dans le flacon désignés par la référence générale 8 et qui est adapté pour permettre le passage d'une pipette d'aspiration de la suspension.

La partie supérieure du flacon désignée par la référence générale 9 sur cette figure 1, est adaptée pour recevoir un couvercle désigné par la référence générale 10.

Des moyens de fixation, comme par exemple des moyens de vissage complémentaires, peuvent être prévus entre cette partie supérieure 9 du flacon et le couvercle 10.

A cet effet, la partie supérieure du flacon est filetée, tandis que la partie correspondante du couvercle 10 est taraudée, ce qui permet d'assurer un vissage du couvercle sur l'extrémité correspondante du flacon.

De cette façon, l'extrémité ouverte du tube 7 est protégée.

De plus, la partie inférieure du flacon comporte un cône de décantation désigné par la référence générale 11.

Les moyens de maintien du tube 7 en position dans le flacon, désignés par la référence générale 8, sont illustrés plus en détail sur la figure 2.

En fait, ces moyens de maintien peuvent comporter des bras de maintien en position s'étendant entre le flacon 1 et le tube 7.

Dans l'exemple illustré, trois bras de maintien disposés sensiblement à 120° l'un par rapport à l'autre, sont représentés.

Bien entendu, d'autres configurations peuvent être prévues.

L'un des bras, comme par exemple le bras 12, peut être muni de moyens de butée pour permettre de détacher de façon classique, une brosse de prélèvement d'un manche de manipulation de celle-ci, afin que cette brosse tombe dans le voile de matériau filtrant, formant panier.

Ces moyens de butée sont, par exemple, formés par une portion en creux 13 du bras.

Ce bras et cette portion en creux peuvent également munis de picots formant moyens de peignage d'une brosse de prélèvement pour faire tomber les éléments prélevés et maintenus sur cette brosse, dans les moyens de filtrage en forme de panier, si l'on ne souhaite pas déposer la brosse dans les moyens formant panier.

On conçoit alors que la suspension peut être aspirée par exemple par une pipette d'aspiration introduite dans le tube 7, ce qui permet de récupérer les différents niveaux de la suspension entre les moyens filtrants et le cône de décantation.

Bien entendu, d'autres modes de réalisation encore peuvent être envisagés.

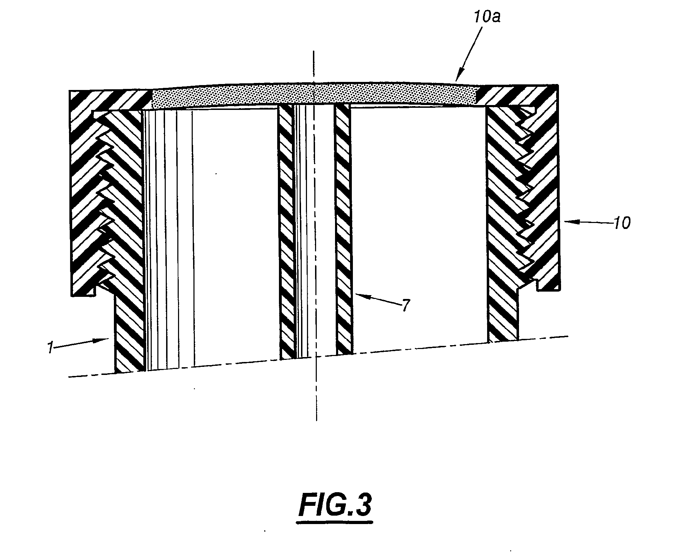

Ainsi, par exemple, on a illustré une variante de réalisation de ce flacon sur la figure 3.

On reconnaît en effet sur cette figure 3, le flacon désigné par la référence générale 1 et son tube désigné par la référence générale 7.

De même, on reconnaît sur cette figure, le couvercle désigné par la référence générale 10 adapté pour être vissé sur l'extrémité correspondante du flacon 1.

Selon l'exemple de réalisation illustré sur cette figure 3, ce couvercle 10 comporte en regard de l'extrémité correspondante du tube, au moins une zone ou portion perçable et auto-cicatrisante de passage de la pipette d'aspiration.

Dans l'exemple illustré, ce couvercle comporte une telle zone qui est désignée par la référence générale 10a prévue par exemple au centre du couvercle 10 et qui permet le passage de la pipette ou de tout autre moyen d'aspiration.

De telles zones perçables et auto-cicatrisantes sont déjà connues en soi dans l'état de la technique.

On ne la décrira donc pas plus en détail par la suite.

On notera également que cette zone est adaptée pour venir, lors du serrage du couvercle sur le reste du flacon 1, en appui contre l'extrémité correspondante du tube 7 afin d'assurer une étanchéité optimale entre ce tube 7 et le reste du flacon, évitant ainsi toute contamination.

Bien entendu, d'autres modes de réalisation encore peuvent être envisagés.

## Revendications

1. Flacon de préparation d'une suspension cytologique à base de fixateur, équipé de moyens (4) de filtrage au moins en partie immergés dans la suspension, **caractérisé en ce que** les moyens de filtrage (4) se présentent sous la forme d'un voile de matériau filtrant, formant panier, dont la périphérie (5) est fixée sur le flacon et dont le centre (6) est raccordé à un tube (7), s'étendant en direction de l'ouverture (2) du flacon, associé à des moyens (8) de maintien en position dans le flacon et adapté pour permettre le passage d'une pipette d'aspiration de la suspension.

2. Flacon selon la revendication 1, **caractérisé en ce que** la partie supérieure (9) du flacon est adaptée pour recevoir un couvercle (10).

3. Flacon selon la revendication 2, **caractérisé en ce que** le couvercle (10) et la partie correspondante (9) du flacon (1) comprennent des moyens de vissage complémentaires.

4. Flacon selon la revendication 2 ou 3, **caractérisé en ce que** le couvercle (10) comporte en regard de l'extrémité correspondante du tube (7), au moins une portion (10a) perçable et auto-cicatrisante, de passage de la pipette d'aspiration, et adaptée pour venir en appui contre cette extrémité du tube (7) afin d'assurer l'étanchéité entre ce tube (7) et le reste du flacon (1).

5. Flacon selon l'une quelconque des revendications précédentes, **caractérisé en ce que** la partie inférieure du flacon (1) comporte des moyens (11) formant cône de décantation.

6. Flacon selon l'une quelconque des revendications précédentes, **caractérisé en ce que** les moyens de maintien (8) du tube (7) dans le flacon comportent des bras de maintien en position, s'étendant entre le flacon (1) et le tube (7).

7. Flacon selon la revendication 6, **caractérisé en ce que** l'un (12) des bras comporte des moyens de butée (13) pour permettre de détacher une brosse de prélèvement d'un manche de manutention afin que celle-ci tombe dans le panier.

8. Flacon selon l'une quelconque des revendications précédentes, **caractérisé en ce que** l'un des bras (12) comporte des picots de peignage d'une brosse de prélèvement pour faire tomber les éléments prélevés par celle-ci dans le panier.

## Claims

1. Flask for preparing a fixer-based cytological solution, equipped with a filtering means (4) at least in the parts immersed in the solution and **characterised in that** the filtering means (4) are in the form of a web of filtering material forming a basket whose periphery (5) is fixed to the flask and whose centre (6) is connected to a tube (7) extending towards the opening (2) of the flask and connected to a means (8) for holding it in position in the flask and adapted to allow the passage of a suction pipette for the solution.

2. Flask according to claim 1, **characterised in that** the upper part (9) of the flask is adapted to receive a cover (10).

3. Flask according to claim 2, **characterised in that** the cover (10) and the corresponding part (9) of the flask (1) comprise complementary screw-threaded means.

4. Flask according to claim 2 or 3, **characterised in that** the cover (10) includes, opposite the corresponding end of the tube (7), at least one portion (10a) that can be pierced and is self-sealing for passage of the suction pipette and adapted to come into contact with said end of the tube (7), in order to ensure the tight seal between said tube (7) and the rest of the flask (1).

5. Flask according to any one of the above claims, **characterised in that** the lower part of the flask (1) comprises means (11) forming a decanting cone.

6. Flask according to any one of the above claims, **characterised in that** the means (8) for holding the tube (7) in the flask comprise arms for holding said tube in position and extending between the flask (1) and the tube (7).

7. Flask according to claim 6, **characterised in that** the one (12) of the arms includes a flange (13) for detaching a sampling brush from a holding sleeve so that it falls into the basket.

8. Flask according to any one of the above claims, **characterised in that** one of the arms (12) includes combing bristles of a sampling brush for causing the elements sampled by it to fall into the basket.

## Patentansprüche

1. Kolben zur Herstellung einer zytologischen Suspension auf der. Basis eines Fixiermittels, versehen mit Filtermitteln (4), die mindestens teilweise in die Suspension getaucht sind, **dadurch gekennzeichnet, dass** die Filtermittel (4) die Form einer Umhüllung aus Filtermaterial aufweisen und einen Korb bilden, dessen Peripherie (5) auf dem Kolben befestigt ist und dessen Zentrum (6) an ein Rohr (7) angeschlossen ist, das sich in Richtung der Öffnung (2) des Kolbens erstreckt, das mit Mitteln (8) zum Halten in Position im Kolben verbunden ist und ausgeführt ist, um den Durchgang einer Pipette zum Absaugen der Suspension zu ermöglichen.

2. Kolben nach Anspruch 1, **dadurch gekennzeichnet, dass** der obere Teil (9) des Kolbens ausgeführt ist, um einen Deckel (10) aufzunehmen.

3. Kolben nach Anspruch 2, **dadurch gekennzeichnet, dass** der Deckel (10) und der entsprechende Teil (9) des Kolbens (1) komplementäre Verschraubungsmittel umfassen.

4. Kolben nach Anspruch 2 oder 3, **dadurch gekennzeichnet, dass** der Deckel (10) hinsichtlich des entsprechenden Endes des Rohrs (7) mindestens einen durchbohrbaren selbstheilenden Abschnitt (10a) zum Durchgang der Absaug-Pipette umfasst und derart ausgeführt ist, dass er auf diesem Ende des Rohrs (7) aufliegt, um die Dichtheit zwischen diesem Rohr (7) und dem Rest des Kolbens (1) zu gewährleisten.

5. Kolben nach einem der vorhergehenden Ansprüche, **dadurch gekennzeichnet, dass** der untere Teil des Kolbens (1) Mittel (11) umfasst, die einen Absetzkegel bilden.

6. Kolben nach einem der vorhergehenden Ansprüche, **dadurch gekennzeichnet, dass** die Mittel (8) zum Halten des Rohrs (7) im Kolben Arme zum Halten in Position umfassen, die sich zwischen dem Kolben (1) und dem Rohr (7) erstrecken.

7. Kolben nach Anspruch 6, **dadurch gekennzeichnet, dass** einer (12) der Arme Anschlagmittel (13) umfasst, um das Lösen eines Probenentnahme-Pinsels von einer Handhabungshülse zu ermöglichen, damit dieser in den Korb fällt.

8. Kolben nach einem der vorhergehenden Ansprüche, **dadurch gekennzeichnet, dass** einer der Arme (12) Kämmnadeln eines Probenentnahme-Pinsels umfasst, um die mit diesem entnommenen Elemente in den Korb fallen zu lassen.
